# EUROPEAN PATENT SPECIFICATION

(11) **EP 0 520 011 B1**
(45) Date of publication and mention of the grant of the patent: **01.03.1995**
(21) Application number: 91906645.6
(22) Date of filing: 12.03.1991
(51) Int. Cl.: C12N 15/00, A61K 39/02, C12N 1/21, A01K 67/02

(54) **VACCINES FOR PREVENTING FURUNCULOSIS IN FISH**
IMPFSTOFFE ZUR SCHUTZIMPFUNG GEGEN FURUNKULOSE BEI FISCHEN
VACCINS POUR LA PREVENTION DE LA FURONCULOSE CHEZ LES POISSONS

(30) Priority: 13.03.1990 IE 88790
(43) Date of publication of application: 30.12.1992
(73) Proprietor: THE PROVOST, FELLOWS AND SCHOLARS OF THE COLLEGE OF THE HOLY AND UNDIVIDED TRINITY OF QUEEN ELIZABETH NEAR DUBLIN, Dublin 2 (IE)
(72) Inventor: FOSTER, Timothy, James, Templeogue, Dublin 16 (IE); VAUGHAN, Laurence, Michael, Drumcondra, Dublin 9 (IE)
(74) Representative: Gates, Marie Christina Esther
(86) International application number: EP9100490
(87) International publication number: WO9113978

(56) References cited:
- WO-A-86/03123
- WO-A-89/05856
- WO-A-89/09063
- WO-A-89/09616
- Infection and Immunity, vol. 58, no. 3, March 1990, American Society for Microbiology, pp. 732-739; M. Roberts et al.
- J. of Fish Diseases, vol. 8, 1985, pp. 43-55; G. Olivier et al.
- Progressive Fish-Cult., vol. 44, no. 4, Oct. 1982, pp. 167-169, R.C. Cipriano et al.

## Description

The present invention relates to attenuated strains of Aeromonas and to use of such strains as vaccines for the prevention of furunculosis in fish. In particular the invention relates to attenuated strains of Aeromonas bearing a mutation in the aromatic biosynthetic pathway.

Aeromonas salmonicida is the causative agent of furunculosis, a disease which can affect a wide variety of bony fish and is of major importance in salmon and trout farming. The disease is generally fatal in salmon and trout and leads to the use of high levels of antibiotics in the fish farming industry, which can have serious environmental effects.

Considerable effort has been directed towards producing an effective vaccine against furunculosis. Most previous vaccines have been bacterins or toxoided subcellular components, or a combination of both (reviewed by Austin and Austin, 1987). Attenuated variants of previously virulent A. salmonicida strains can easily be derived by subculture in the laboratory (McCarthy and Roberts, 1980; Munn and Trust, 1984). This attenuation is usually associated with loss of the outer surface protein A-layer. A⁻strains are unsuitable as live-attenuated vaccines because they lack the surface antigenic determinants associated with virulent A⁺ strains (McCarthy et al, 1983; Olivier et al, 1985) and they persist poorly when administered to fish (Munn and Trust, 1984). However, one such strain was reported to elicit protective immunity in Atlantic salmon (Cipriano and Starliper, 1982), but has not been developed into a commercial vaccine.

Attenuated strains which retain the A layer have been described (Hackett et al, 1984; Sakai, 1985) but the exact nature of the attenuation was unknown. If a point mutation was involved the variant could revert to virulence following administration to fish. A protease-deficient mutant isolated following chemical mutagenesis did not elicit protective immunity (Sakai, 1985). Thus previous live-attenuated strains have been unsuitable for vaccination against furunculosis (Michel, 1982).

According to the present invention there is provided an attenuated strain of Aeromonas bearing a mutation in a gene of the aromatic biosynthetlc pathway, the strain being deficient in PABA synthesis.

Preferably, the mutation is non-reverting so that it cannot be repaired in a single step. The mutation may be an inversion, substitution or deletion mutation.

In particular, the mutation may be in the aroA gene (i.e. the gene encoding 5-enolpyruvylshikimate-3-phosphate (EPSP) synthase).

The invention also relates to the Aeromonas salmonicida strain DU 5847 deposited at the National Collection of Industrial and Marine Bacteria, 23, St. Machar Drive, Aberdeen AB2 1RY, Scotland under the Accession No. 40261 on 26th February 1990, or to such a strain having the kanamycin drug sensitivity marker excised, or strain DU5860 deposited at the National Collection of Industrial and Marine Bacteria, 23, St. Machar Drive, Aberdeen AB2 1RY, Scotland under the Accession No. NCIMB 40381 on 11th March 1991 ,or to strains of Aeromonas substantially similar to the deposited strains in the region of DNA encoding a mutant gene of the aromatic biosynthetic pathway, the said strain being attenuated.

Aromatic-dependent mutants of the present invention offer considerable improvements over previous live-attenuated vaccines for the following reasons:
(i) The cause of the attenuation is precisely defined.
(ii) The attenuated bacterium retains surface antigens present on the virulent organism and is thus a good immunogen.
(iii) The attenuated organisms are completely avirulent.
(iv) The Aro⁻ phenotype cannot revert.

By "substantially similar" herein is meant strains of A. salmonicida which have sufficient sequence identity or homology to the deposited strains in the region of the mutant gene of the aromatic biosynthetic pathway to hybridize therewith and to be deficient in PABA synthesis and to be attenuated, and also includes strains which have deletions of the entire relevant gene of the aromatic biosynthetic pathway, which are also deficient in PABA synthesis and are attenuated.

The invention also relates to the use of an attenuated Aeromonas strain bearing a mutation in a gene of the aromatic biosynthetic pathway in the preparation of a vaccine for the prevention of furunculosis.

In a further aspect the invention provides a vaccine against furunculosis comprising an attenuated strain of Aeromonas bearing a mutation in a gene of the aromatic biosynthetic pathway.

The invention further provides a method of prevention of furunculosis in fish comprising immunising fish with an attenuated strain of Aeromonas bearing a mutation in a gene of the aromatic biosynthetic pathway in an amount sufficient to elicit an immune response. Preferably the method comprises administering a first immunising amount followed by a second booster amount of the said attenuated Aeromonas strain.

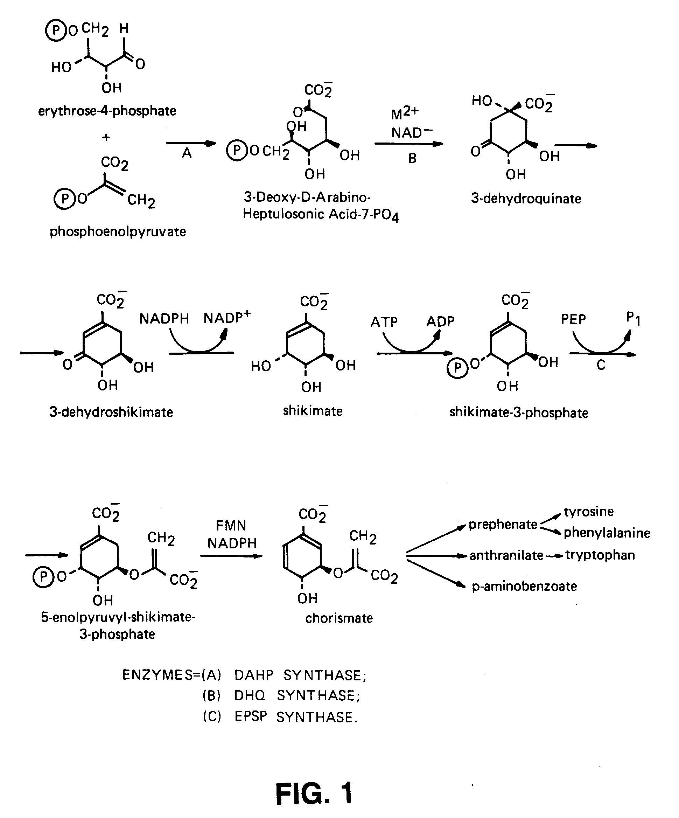

The biosynthetic pathway for aromatic compounds leads initially from the reaction of erythrose-4-phosphate and phosphoenolpyruvate to the eventual synthesis of chorismic acid. The pathway then branches forming aromatic amino acids and p-aminobenzoic acid (PABA) which in turn is converted to folic acid, a co-factor with a crucial role in biosynthesis of DNA and proteins (Fig. 1). In contrast to bacteria, higher animals do not synthesise folic acid or PABA. Bacteria which are deficient in PABA synthesis cannot grow in infected animals because exogenous PABA does not occur at sufficiently high levels to allow the metabolic block to be by-passed and in addition bacteria cannot take up exogenous folic acid.

Aromatic biosynthetic pathway mutants of other species of bacteria are known but it could not have been predicted that such mutants of Aeromonas would have been sufficiently attenuated not to cause disease symptoms in infected fish and at the same time would persist in the fish for long enough to induce an immune response. For example, S. typhimurium aroA mutants were found to be effective live vaccines in mice against murine salmonellosis (Hoiseth & Stocker 1981), and S. typhimurium strains harbouring mutations in each of two aromatic biosynthetic genes were found to be effective as vaccine strains against murine salmonellosis (PCT Publication No. PCT/GB88/01143 of The Wellcome Foundation Ltd.). However, of three avirulent Salmonella typhimurium aromatic-dependent mutants, two were found to be ineffective as live-organism vaccines in calves (Smith et al, 1984). In addition aroA mutants of Yersinia enterocolitica gave poor protection against experimental infection in rats (Bowe et al., 1989) and aroA mutants of Salmonella choleraesuis were not effective as live vaccines in mice (Nnalue & Stocker, 1987). However, it has surprisingly been found that aromatic-dependent mutants of Aeromonas salmonicida are avirulent, survive for up to about two weeks in infected fish and stimulate strong protective immunity. They are therefore suitable for use as live attenuated vaccines against furunculosis. Such vaccines also have the advantage that the bacteria are presented in an effective way to the host's immune system.

The present invention will now be described in greater detail with reference to the accompanying drawings, in which:-
Figure 1 is a summary of the biosynthetic pathway for the biosynthesis of aromatic compounds.

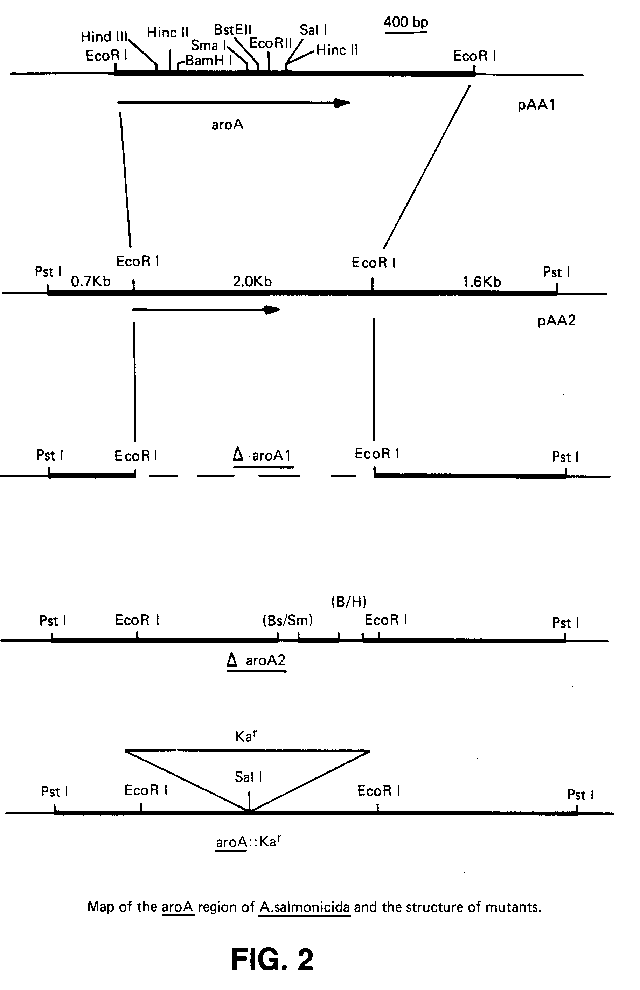

Figure 2. Map of aroA region of A. salmonicida and structure of mutants. The upper part of the figure shows a restriction map of the 4.3 kb Pst1 fragment which carries aroA. A more detailed map of the expanded 2.0 kb EcoRI fragment is given. The horizontal arrow shows the extent of the aroA gene derived from DNA sequencing, in the direction of transcription. The triangle indicates the position of the insertion in aroA by the Ka^{r} fragment in aroA::Ka^{r}. Also shown are two deletions, ΔaroA1 and ΔaroA2. The restriction sites are abbreviated as follows: P=PstI; E=EcoRI; Hc=HincII; X=XhoI; RV=EcoRV; Sa=SalI Bs=BstEII; B=BamHI; H=HindIII.
Figure 3. Isolation of A. salmonicida aroA::Ka^{r}
   The diagram depicts the steps in the isolation of the replacement mutation aroA::Ka^{r} in A. salmonicida 644Rb. The circle represents the suicide plasmid pSUP202 carrying the aroA::Ka^{r} mutation (details in Figure 2). The box is the aroA gene and the triangle the Ka^{r} insertion into the SalI site. A single Campbell-type cross-over occurs between homologous sequences in the aroA region allowing the plasmid to integrate into the chromosome. The cells become resistant to kanamycin, tetracycline and chloramphenicol and remain Aro⁺. Growth in drug free broth allows the plasmid to excise. Some excisants remain Ka^{r} if the second cross-over occurs on the other side of the Ka^{r} insertion. The mutants are detected by their Aro⁻ Ka^{r} phenotype.

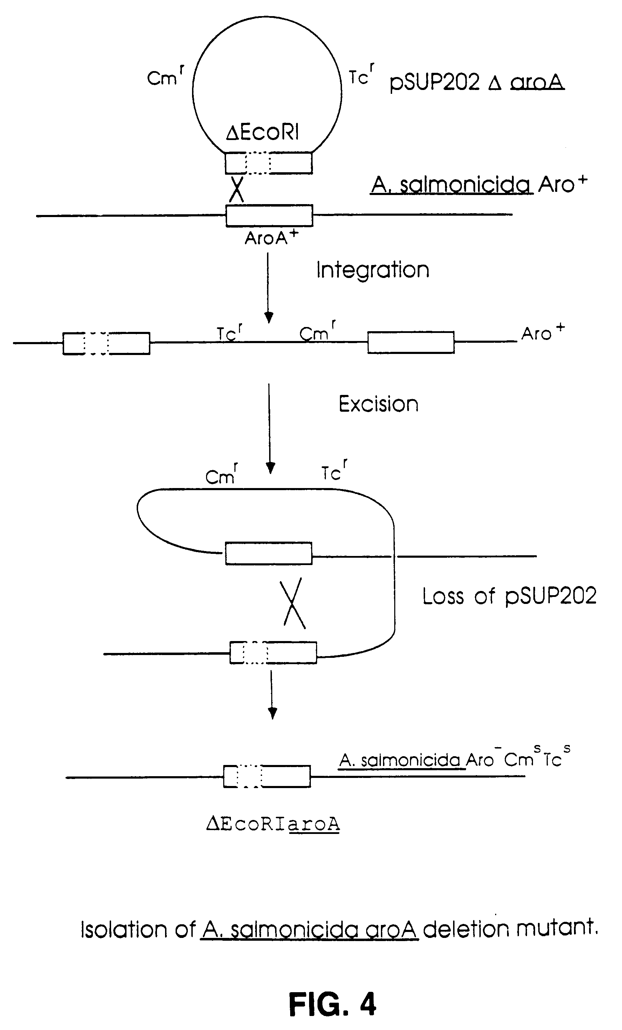

Figure 4. Isolation of A. salmonicida ΔaroA
   The diagram shows the steps involved in the isolation of a deletion mutation in the aroA gene of A. salmonicida by allele replacement. The circle represents pSUP202 carrying the deleted aroA gene indicated by the short box with the dotted outline. The plasmid integrates into the chromosome by a single Campbell-type recombination event at the homologous aroA locus (longer box). The cells are Aro⁺ Tc^{r} Cm^{r}. After growth in drug-free broth, plasmid excision occurs resulting in drug sensitive cells. If the excision event occurs on the opposite side of the deletion to the insertion event the cell becomes Aro⁻.

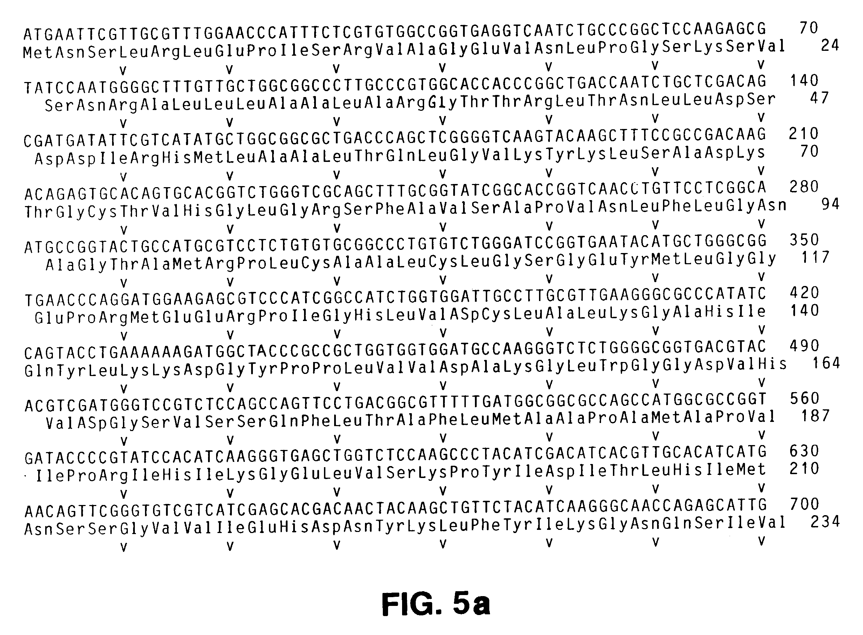

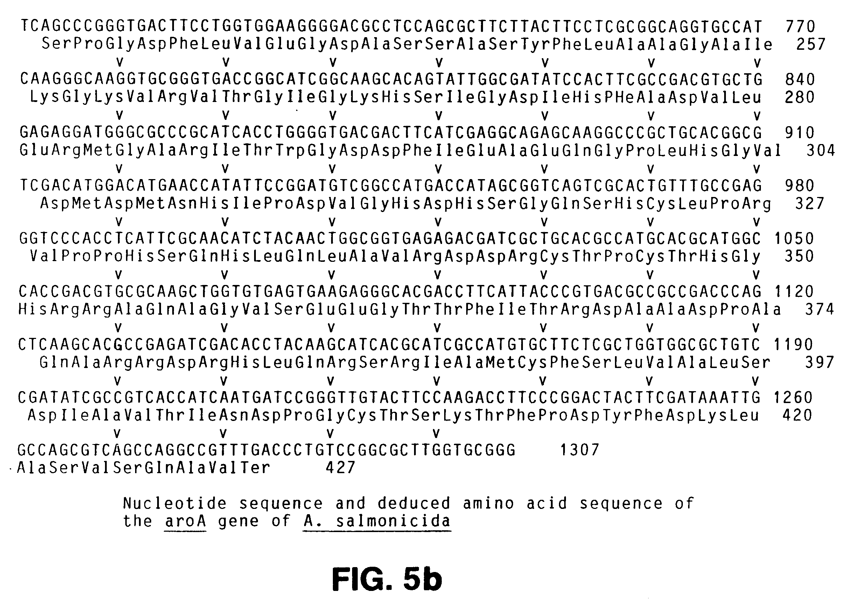

Figure 5. Nucleotide sequence and deduced amino acid sequence of the aroA gene of A. salmonicida.

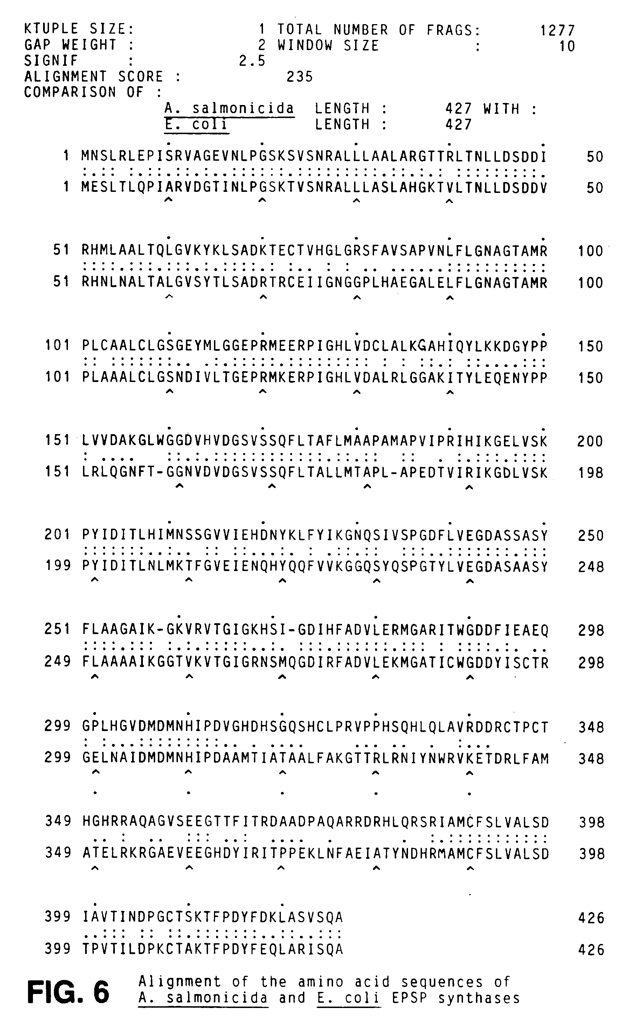

Figure 6. Wilbur alignment of the amino acid sequences of A. salmonicida and E. coli EPSP synthases. : =indentical amino acid residues. . = conservative amino acid substitutions.

### Construction of aromatic-dependent mutants of Aeromonas salmonicida.

### Example 1 -An aro mutation associated with antibiotic resistance.

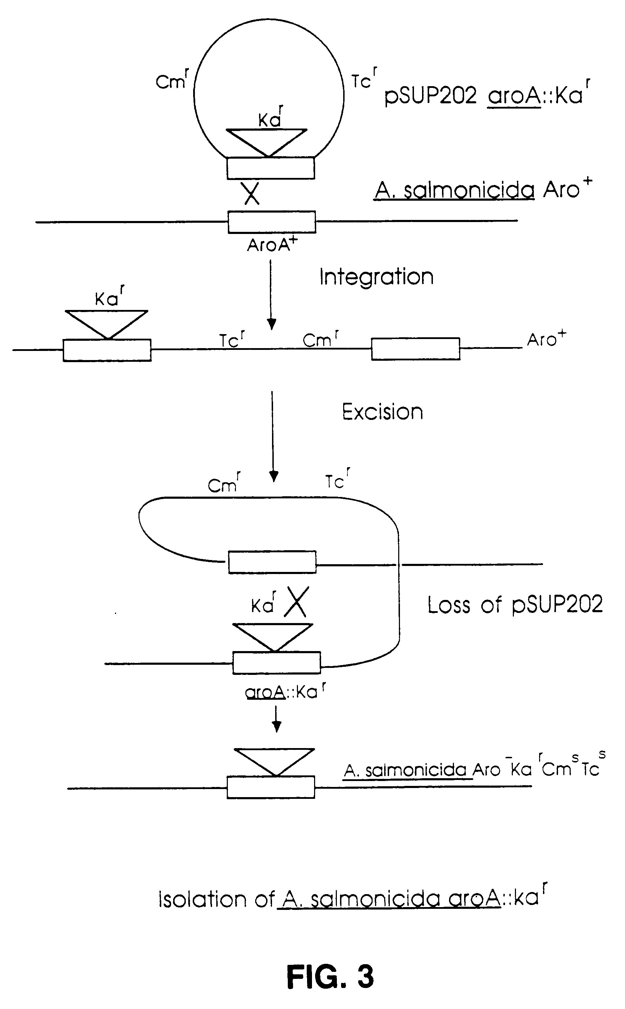

The aroA gene of A. salmonicida was cloned on a plasmid vector in E.coli AB1321 selecting for complementation of the aroA mutation (O'Reilly et al, 1988). The gene was mapped to a 2 kb EcoRI fragment (Figure 2). The cloned aroA gene was inactivated by inserting a fragment of DNA expressing kanamycin resistance into the centrally located SalI site (Figure 2,3). This plasmid carrying the aroA::Ka^{r} construct was no longer able to complement E. coli AB1321.

In order to facilitate high frequency homologous recombination with the aroA locus in the A. salmonicida chromosome the aroA::Ka^{r} mutation was constructed with longer flanking sequences (Figure 2).

This process may be described in greater detail as follows:-
The aroA gene was cloned from genomic A. salmonicida DNA in a bacteriophage λ vector and in plasmid vectors using standard procedures (Sambrook et al, 1989).

High molecular weight genomic DNA of A. salmonicida was cleaved partially with Sau3A1. Fragments of 15-20 kb were separated by centrifugation in a sucrose gradient. About 1 »g was ligated with 1»g of the λ replacement vector λ2001 (Karn et al, 1984) cleaved with BamHI. The DNA was packaged in vitro (Hohn, 1979) and the reconstructed phage particles were plated with a lawn of Escherichia coli AB1321 aroA in minimal medium lacking aromatic supplements. Phage carrying the A.salmonicida aroA gene were recognised by their ability to form plaques surrounded by a zone of enhanced growth of the lawn (O'Reilly et al, 1988).

Pst1 and EcoRI fragments of aroA were cloned into plasmids pUC18 (Yanish-Perron et al, 1985) and pBR322 (Bolivar et al, 1977) and Aro⁺ derivatives were isolated by transforming into AB1321 selecting for Tc^{r} or Ap^{r} colonies as appropriate and then replica-plating to detect complementation. Restriction mapping (Figure 2) revealed that the 2 kb complementing EcoRI fragment was contained within a 4.3kb PstI fragment.

The cloned aroA gene carried by the PstI fragment was inactivated by inserting a 2.2kb SalI-XhoI fragment which carries the kanamycin resistance gene from transposon Tn5 (Putnoky et al, 1983), into the centrally located SalI site (Figure 2). This plasmid was no longer able to complement AB1321.

The aroA::Ka^{r} cassette was cloned into the PstI site in the broad host range suicide plasmid pSUP202 (Simon et al, 1983). The chimeric pSUP202aroA::Ka^{r} plasmid was transformed into the mobilizing E.coli donor strain S17-1 (Simon et al, 1983). Cells from an overnight broth culture of E.coli S17-1 pSUP202aroA::Ka^{r} (grown in L Broth at 37^{o}C) were mixed with a 48h culture of freshly passaged (see Example 3 below) A. salmonicida 644Rb (resistant to nalidixic acid) grown at 22^{o}C in Trypticase Soy Broth (TSB). 0.02 ml of the E.coli donor culture was mixed with 0.1ml of the A. salmonicida culture on a 0.45 micron nitrocellulose filter on the surface of a Trypticase Soy Agar (TSA) plate which was incubated at 30^{o}C for 4h. The cells were harvested, resuspended in saline and plated onto selective plates (TSA with kanamycin and nalidixic acid at 40»g/ml and 30»g/ml, respectively).

Transconjugants were recovered at a frequency of 10⁻² per recipient. Ka^{r} can only be inherited by A. salmonicida following recombination with the chromosome at the aroA locus. The most frequent event is a single Campbell-type cross-over (Figure 3). More rarely (10⁻⁴ per recipient) double recombinants were obtained (Figure 3). The recombinants were recognised by their phenotypes (summarized in Figure 3). The structure of the recombinants was verified by DNA hybridization (Southern, 1975), using the cloned A. salmonicida aroA gene as a probe.

One of these recombinants, A. salmonicida strain DU5847, was deposited at the National Collection of Industrial and Marine Bacteria, 23 St. Machar Drive, Aberdeen AB2 IRY, Scotland on 26th February 1990 under the accession No. 40261.

### Example 2 - aroA deletion mutations

The aroA gene of A. salmonicida has been sequenced, facilitating the construction of deletions within the coding sequence which will completely inactivate aroA and which will be non-revertible. Two deletion mutants have been constructed on plasmids: ΔaroA1 is a large deletion which removes all the coding sequence located between the EcoR1 sites. ΔaroA2 suffers two smaller deletions within the aroA coding sequence (Figure 2). These two mutants can be used to construct aroA mutants of any Aeromonas strain.

The sequence of the aroA gene A. salmonicida is shown in Figure 5. The amino acid sequence of the putative EPSP synthase was deduced from the DNA sequence (Fig. 5). It is very similar to the extensively characterised E. coli E. coli EPSP synthase (Fig. 6).

The ΔaroA mutations in A. salmonicida can be constructed using the pSUP202 suicide vector. They can be introduced into the A. salmonicida chromosome to replace the wild-type aroA gene (Figure 4). The desired recombinants can be detected by changes in phenotype (Aro⁺ to Aro⁻) and verified by DNA hybridization. This technique can be used to remove the Ka^{r} marker from the deposited A. salmonicida strain DU5847 to produce a strain devoid of drug resistance markers and suitable for use as a commercial vaccine.

The ΔaroA1 deletion was constructed by removing the 2kb EcoRI fragment from a plasmid carrying the larger PstI fragment (Fig. 2). The PstI fragment with ΔaroA1 was then cloned into the PstI site in the mobilizable suicide plasmid pSUP202 (Simon et al 1983).

The plasmid was transferred into A. salmonicida Aro⁺ using the plate-mating procedure previously described. Growth on tetracycline selected recombinants where the plasmid was integrated onto the chromosome by a single cross-over (Fig. 4). These recombinants were resistant to tetracycline and to chloramphenicol and were Aro⁺. Growth in non-selective broth allowed derivatives which were Tc⁻Cm⁻Aro⁻ to be identified. The structure of the aro locus of A. salmonicida ΔaroA1 was verified by Southern hybridization (Southern, 1975) using the aroA gene as a probe. This strain does not revert to Aro⁺ and is devoid of drug resistance markers. It is suitable for use as a commercial vaccine.

This A. salmonicida strain DU5860 bearing the ΔaroA1 mutation was deposited at the National Collection of Industrial and Marine Bacteria, 23 St. Machar Drive, Aberdeen AB2 IRY, Scotland on 11th March 1991 under the accession No. NCIMB 40381.

It will be understood by one skilled in the art that these techniques could also be used to produce an A. salmonicida strain bearing the ΔaroA2 mutation as well as mutants in genes of the aromatic synthetic pathway other than the aroA gene.

### Example 3 - Assessment of vaccine potential.

### Virulence

It is well know that A. salmonicida losses its virulence on prolonged laboratory subculture (McCarthy and Roberts, 1980). Accordingly the aroA::Ka^{r} mutation was introduced into a known virulent strain, 644Rb which had been freshly passaged in vivo. This was achieved by intramuscular (im) injection of bacteria into a brown trout and subsequently recovering the organism from the kidney post-mortem on four successive occasions. This strain had an LD₅₀ of <10² cells.

The mutants were then introduced into this virulent strain with the minimum number of laboratory subcultures in order to preserve protective antigens as follows:
(i) The virulent strain was obtained by streaking the contents of a kidney from an infected fish on a TSA plate.
(ii) A single colony was inoculated into TSB. This culture was used directly in mating with E.coli carrying the suicide plasmid.
(iii) Dilutions of the mating mixture were plated on TSA plates with selective antibiotics.
(iv) A single transconjugant colony was replated once as a dense sweep. (The phenotype was checked by cross-streaking and mutants were identified).
(v) The sweep growth was streaked for single colonies.
(vi) A single colony was inoculated into TSB. This culture was stored in aliquots at -80^{o} after snap-freezing in liquid nitrogen.
(vii) For virulence studies and for preparation of the immunogen, ice from the top of the frozen culture was streaked on a TSA plate for single colonies.
(viii) A colony was inoculated into TSB. This culture provided the inoculum for virulence studies and vaccination.

The retention of virulence factors of Aro⁻ mutants of A. salmonicida 644Rb was established by transferring the wild-type aroA gene into the mutants on a broad host range plasmid pGSS33 (Sharpe, 1984). Injection of 10⁷ cells im resulted in 75% mortalities from furunculosis.

### Persistence

In order to assess persistence in vivo, 40 brown trout were inoculated with 10⁷ Aro⁻ bacteria and samples were taken at daily intervals. Bacteria were found in the kidneys (a site where large numbers are found in fish infected with virulent organisms) at about 10³ cfu (colony forming units per kidney) up to 10 days post infection but none were isolated after 14 days. Clearly the bacteria can persist in the host without causing symptoms. This is probably necessary to stimulate protective immunity.

### Immune response

Serum antibody levels in fish which were injected IP with Aro⁻ bacteria were measured by agglutination titration. The antibody titre rose from < 2 to 128 after 6 weeks. This shows that injection with Aro⁻ bacteria stimulates an immune response.

### Protection

Brown trout were injected intraperitoneally with 10⁷ Aro⁻ bacteria to stimulate immunity. Six weeks after the initial vaccination 20 fish were given a booster injection with the same number of avirulent bacteria. Groups of 4 vaccinated and unvaccinated fish were then challenged with virulent organisms ranging from 10¹ - 10⁷ cfu. The number of deaths and the time of death of challenged fish were recorded and the LD₅₀ calculated.

| | |
|---|---|
| Unvaccinated control | LD₅₀ 7.2 x 10¹ cfu |
| Vaccinated | LD₅₀ 1.8 x 10⁴ cfu |
| Vaccinated & Boosted | LD₅₀ 3.0 x 10⁵ cfu |

This data clearly demonstrates that the Aro⁻ Aeromonas salmonicida lacks virulence for salmon and trout and that injecting the bacteria intra-peritoneally results in significant protective immunity.

### Example 4 - Stability Study.

The long term stability of the A. salmoncida 644Rb Aro⁻ mutant strain was assessed in a number of routine stabilizing media over a period of months. An overnight A. salmonicida culture was centrifuged, washed and the cells resuspended in one of three stabilizing media or nutrient broth. The suspensions were then lyophilised and stored at room temperature. At various times after lyophilisation the cells were resuspended in water and counted. The stabilizing media comprise (A) 7.5% sucrose, 2% sodium glutamate, 4.5% dextran, (B) 10% sucrose, 1% sodium glutamate, 5% dextran, (C) 10% sucrose, 1% sodium glutamate; all weight to volume percentages in water and (D) broth control. The results of the viability test are shown in Table I, indicating that the strain showed adequate stability over the period tested.

**TABLE I**

| Stabilizing Medium | Viable Count (cfu/m1) | | | |
|---|---|---|---|---|
| | tₒ | 1mth | 2mth | 3mth |
| A. | 1.09 x 10¹⁰ | 9.25 x 10⁷ | 1.895 x 10⁸ | 1.145 x 10⁸ |
| B. | 6.75 x 10⁸ | 3.3 x 10⁸ | 4.55 x 10⁸ | 2.145 x 10⁸ |
| C. | 6.2 x 10⁹ | 2.4 x 10⁹ | 1.86 x 10⁹ | 3.5 x 10⁸ |
| D. | 1.21 x 10⁸ | 1.48 x 10⁸ | 4.65 x 10⁷ | 2.25 x 10⁶ |

### References

Austin, B., and Austin, D.A. (1987) Aeromonas Chapter 9. In: Bacterial fish pathogens-disease in farmed and wild fish, Ellis Horwood Chichester, pp. 111-195.

Bolivar, F., Rodriquez, R.L., Greene, P.J., Betlach, M.C., Heyneker, H.L., Boyerm H.W., Crosa, J.H. and Falkow, S. (1977) Construction and characterization of new cloning vehicles. III. A multipurpose cloning system. Gene. 2:95-113.

Bowe, F., O'Gara, P., Maskell, D., Cafferkey, M. and Dougan, G. (1989) Virulence, persistence, and immunogenicity of Yersinia enterocolitica 0:8 aroA mutants. Infection and Immunity 57: 3234-3236.

Cipriano, R.C. and Starliper, C.E. (1982) Immersion and injection vaccination of salmonids against furunculosis with an avirulent strain of Aeromonas salmonicida. Progressive Fish Culturist 44::167-169.

Hackett, J.L., Lynch, W.H., Paterson, W.D. and Combs, D.H. (1984). Extracellular protease, extracellular haemolysin and virulence in Aeromonas salmonicida. Can. J. Fish. Aquat. Sci. 41: 1354-1360.

Hohn, B. (1979). In vitro packaging of lambda and cosmid DNA. Methods in Enzymology 68:299-309.

Hoiseth, S.K. and Stocker B.A.D. 1981. Aromatic-dependent Salmonella typhimurium are non-virulent and effective as live vaccines. Nature (London) 291 : 238-239

Karn, J., Matthes, H.W.D., Gait, M.J. and Brenner, S. (1984). A new selective phage cloning vector 2001, with sites for XbaI, BamHI, HindIII, SstI and XhoI. Gene 32:217-224.

McCarthy, D.H. and Roberts, R.J. (1980) Furunculosis of fish - the present state of our knowledge. In: Droop, M.A., and Jannasch, H.W. (eds.), Advances in Aquatic Microbiology, London, Academic Press, pp 293-341.

McCarthy, D.H., Amend, D.F., Johnson, K.A. and Bloom, J.V. (1983) Aeromonas salmonicida: determination of an antigen with protective immunity and evaluation of an experimental bacterin. Journal of Fish Diseases 6: 155-174.

Michel, C. (1982) Progress toward furunculosis vaccination. In Microbial Diseases of Fish (ed. Roberts, R.J.) Academic Press, London, New York. pp. 151-169.

Munn, C.B. and Trust, T.J. (1984) Role of additional protein in virulence of Aeromonas salmonicida. In Fish Diseases, Fourth COPRAQ Session (ed Acuigrup) Editora ATP, Madrid, Spain pp. 69-75.

Nnalue, N.A. & B.A.D. Stocker. (1987) Tests of the virulence and live-vaccine efficacy of auxotrophic and galE derivatives of Salmonella choleraesuis. Infection and Immunity. 55, 955-962.

Olivier, G., Evelyn, T.P.T. and Lallier, R. (1985) Immunogenicity of vaccines from a virulent and an avirulent strain of Aeromonas salmonicida. Journal of Fish Diseases 8: 43-55.

O'Reilly, M., O'Toole, P.W. and Foster, T.J. (1988) Screening lambda libraries and detection of recombinants. In: Owen, P. and Foster, T.J. (eds.), Immunochemical and molecular genetic analysis of bacterial pathogens, Amsterdam, Elsevier, pp. 187-197.

Putnoky, P., Kiss, G.B., Ott, I. and Konorosi, A. (1983). Tn5 carries a streptomycin resistance determinant downstream from the kanamycin resistance gene. Mol. Gen. Genet. 191:288-294.

Sakai, D.K. (1985) Efficacy of specific antibody against agglutinating Aeromonas salmonicida strain on infectivity and vaccination with inactivated protease. Journal of Fish Diseases 8:397-405.

Sambrook, J., Fritsch, E.F. and Maniatis, T. (1989). Molecular cloning. A laboratory manual. 2nd edition. Cold Spring Harbour Laboratory Press, Cold Spring Harbour, New York.

Sharpe, G.S. (1984) Broad host range cloning vectors for gram negative bacteria. Gene 29: 93-102.

Simon, R., Priefer, U. and Puhler, A. (1983) A broad host range mobilization system for in vivo genetic engineering: transposon mutagenesis in gram-negative bacteria. Bio/Technology 1: 784-791.

Smith, B.P., M. Reina-Guerra, S.K. Hoiseth, B.A.D. Stocker, F. Habasha, E. Johnson & F. Merritt., "Aromatic-dependant Salmonella typhimurium as modified live vaccines in calves.", Am. J. Vet. Res. 45, 59-66, 1984.

Southern, E.M. (1975) Detection of specific sequences among DNA fragments separated by gel electrophoresis. J. Mol. Biol. 98: 503-517.

Yanisch-Perron, C., Vieira, J.C., and Messing. J. (1985). Improved M13 phage cloning vectors and host strains: nucleotide sequences of the M13mp18 and pUC19 vectors. Gene. 33:103-119.

## Claims

1. An attenuated strain of Aeromonas bearing a mutation in a gene of the aromatic biosynthetic pathway, the strain being deficient in PABA synthesis.

2. An attenuated strain of Aeromonas as claimed in claim 1 wherein the mutation is non-reverting.

3. An attenuated strain of Aeromonas as claimed in claim 2 wherein the mutation is in the aroA gene.

4. Aeromonas salmonicida strain DU5847 deposited at the National Collection of Industrial and Marine Bacteria, Scotland, United Kingdom under the Accession No. 40261 on 26th February 1990 or an Aeromonas strain substantially similar thereto in the region of DNA encoding a mutant gene of the aromatic biosynthetic pathway, the said strain also being attenuated.

5. Aeromonas salmonicida strain DU5847 as deposited at the National Collection of Industrial and Marine Bacteria, Scotland, United Kingdom under the Accession No. 40261 on 26th February 1990 with the kanamycin drug sensitivity marker excised.

6. Aeromonas salmonicida strain DU5860 deposited at the National Collection of Industrial and Marine Bacteria, Scotland, United Kingdom under the Accession No. NCIMB 40381 on 11th March 1991 or an Aeromonas strain substantially similar thereto in the region of DNA encoding a mutant gene of the aromatic biosynthetic pathway, the said strain also being attenuated.

7. Use of an attenuated Aeromonas strain as claimed in any one of claims 1 to 6 in the preparation of a vaccine for the prevention of furunculosis.

8. A vaccine against furunculosis comprising an attenuated strain of Aeromonas as claimed in any one of claims 1 to 6.

## Patentansprüche

1. Attenuierter Stamm von Aeromonas mit einer Mutation in einem Gen des Aromaten-Biosynthesewegs, wobei der Stamm bezüglich der PABA-Synthese defizient ist.

2. Attenuierter Stamm von Aeromonas nach Anspruch 1, wobei die Mutation nicht-revertierend ist.

3. Attenuierter Stamm von Aeromonas nach Anspruch 2, wobei sich die Mutation im aroA-Gen befindet.

4. Aeromonas salmonicida Stamm DU5847, hinterlegt am 26. Februar 1990 bei der National Collection of Industrial and Marine Bacteria, Schottland, Vereinigtes Königreich, unter der Hinterlegungs-Nr. 40261, oder ein Stamm von Aeromonas, der demgegenüber in der DNA-Region im wesentlichen gleich ist, die für ein mutiertes Gen des Aromaten-Biosynthesewegs kodiert.

5. Aeromonas salmonicida Stamm DU5847, hinterlegt am 26. Februar 1990 bei der National Collection of Industrial and Marine Bacteria, Schottland, Vereinigtes Königreich, unter der Hinterlegungs-Nr. 40261, wobei der Marker für die Kanamycinresistenz herausgeschnitten ist.

6. Aeromonas salmonicida Stamm DU5860, hinterlegt am 11. März 1991 bei der National Collection of Industrial and Marine Bacteria, Schottland, Vereinigtes Königreich, unter der Hinterlegungs-Nr. NCIMB 40381, oder ein Stamm von Aeromonas, der demgegenüber in der DNA-Region im wesentlichen gleich ist, die für ein mutiertes Gen des Aromaten-Biosynthesewegs kodiert, wobei jener Stamm ebenfalls attenuiert ist.

7. Verwendung eines attenuierten Stammes von Aeromonas nach einem der Ansprüche 1 bis 6 zur Herstellung eines Impfstoffes zur Vorbeugung gegen Furunkulose.

8. Impfstoff gegen Furunkulose, umfassend einen attenuierten Stamm von Aeromonas nach einem der Ansprüche 1 bis 6.

## Revendications

1. Souche atténuée d'*Aeromonas* portant une mutation dans un gène de la voie de biosynthèse aromatique, la souche étant déficiente dans la synthèse de PABA.

2. Souche atténuée d'*Aeromonas* suivant la revendication 1, dans laquelle la mutation n'est pas réversible.

3. Souche atténuée d'*Aeromonas* suivant la revendication 2, dans laquelle la mutation est dans la gène aroA.

4. Souche DU5847 d'*Aeromonas salmonicida* déposée à la National Collection of Industrial and Marine Bacteria, Ecosse, Royaume Uni, sous le numéro d'accès No 40261 le 26 février 1990, ou souche d'*Aeromonas* pratiquement similaire à celle-ci dans la région de l'ADN codant pour un gène mutant de la voie de biosynthèse aromatique, cette souche étant aussi atténuée.

5. Souche DU5847 d'*Aeromonas salmonicida* telle que déposée à la National Collection of Industrial and Marine Bacteria, Ecosse, Royaume Uni, sous le numéro d'accès No 40261 le 26 février 1990, dans laquelle le marqueur de sensibilité au médicament kanamycine est excisé.

6. Souche DU5860 d'*Aeromonas salmonicida* déposée à la National Collection of Industrial and Marine Bacteria, Ecosse, Royaume Uni, sous le numéro d'accès No NCIMB 40381 le 11 mars 1991, ou souche d'*Aeromonas* pratiquement similaire à celle-ci dans la région de l'ADN codant pour un gène mutant de la voie de biosynthèse aromatique, cette souche étant aussi atténuée.

7. Utilisation d'une souche d'*Aeromonas* atténuée suivant l'une quelconque des revendications 1 à 6, dans la préparation d'un vaccin pour la prévention de la furonculose.

8. Vaccin contre la furonculose, comprenant une souche atténuée d'*Aeromonas* suivant l'une quelconque des revendications 1 à 6.
